# EUROPEAN PATENT APPLICATION

(11) **EP 1 820 910 A2**
(43) Date of publication of application: **22.08.2007**
(21) Application number: 07075142.5
(22) Date of filing: 19.02.2007
(51) Int. Cl.: E03B 7/09

(54) **Flushing pipes**

(30) Priority: 20.02.2006 NL 1031187; 08.11.2006 NL 1032837
(71) Applicant: Markman, Hendricus, 2435 NG Zevenhoven (NL); Aquador BV, 4221 LL Hoogblokland (NL)
(72) Inventor: Markman, Hendricus, 2435 NG Zevenhoven (NL)
(74) Representative: Assendelft, Jacobus H.W.

(57) **Abstract**

The invention relates to a flushing method in a tap water pipe system, comprising pipes for hot (3) and cold (2) water, connected to tap points (8, 26) to dispense hot and/or cold water. Flushing is carried out by applying a pressure change in the water pipe system or part of it. Then the hot and cold water pipes (2, 3) are short circuited at the tap points to allow flow from the one to the other pipe. Flushing is carried out first at the one pressure level and then at a higher pressure level to activate additional tap points (26). During flushing and post-rinsing, the pressure within the one pipe (3) is lower then the pressure within the other pipe (2). In this way hot water from the boiler (7) can flow first through the cold water pipe (2) and then through the hot water pipe (3) and then back to the boiler (7) to kill bacteria causing Legionnaire's disease, without water consumption.

## Description

The object of the invention is, i.a., counteracting legionella bacteria, particularly in sanitary installations, by flushing the pipes. The invention can however also be used to flush pipes for different purposes or to replace the medium in a pipe, e.g. during batch type production. Although the invention is hereafter disclosed in connection with Legionnaire's disease, it will be appreciated that the invention has also different applications.

As is known, the bacteria causing Legionnaire's disease, grows at temperatures between approximately 25 and 55 degrees Celsius. Above that, it dies, below that, it is inactive. Particularly sanitary cold water pipes in warm, crowded buildings that are used relatively infrequent provide risks.

Many proposals have been made already for counteracting these bacteria. In most cases they are unfit for existing sanitary installations, e.g. since modifications are impossible or costly.

According to a presently frequently applied technique, the pipes are manually flushed with hot water. In e.g. a swimming pool, hospital or residence, hot water is injected into the cold water pipe while all taps of showers, sinks, baths, etc. are opened. One the one hand this is labour intensive. Apart from that, the water consumption is high. There is further the possibility that bacteria are released during flushing. In most cases a specially designed boiler is required. To keep the investment costs at a manageable level, one of the proposals is to apply a mobile boiler, which can thus be used for many buildings. To keep the total costs as low as possible, flushing is kept to a minimum, e.g. once a week or month.

Different techniques that are presently used are internal heating by an electrical wire, or draining of the pipe. These also have disadvantages, e.g. safety, reliability, applicability, costs, operating convenience, use of materials, level of investment, frequency of use, etc.

The object of the invention is versatile and is in a first aspect a different manner of flushing a pipe. This for e.g. sanitary installations. One of the more important aspects that are preferred is to be able to flush with high frequency, e.g. daily or several times a day, against low costs and effort in an environmental friendly manner.

According to the invention a pressure change within the pipe is applied, wherein preferably care is taken that the pressure within the pipe remains above the (e.g. atmospheric) pressure prevailing externally from the tube. The flushing medium can at the by the pressure change caused, increased or decreased pressure, or a with respect to that higher or lower pressure, treat the pipe internally.

E.g. the pressure change is caused by means of opening a closing means, such as a valve. Preferably the flushing medium is supplied to the inside of the pipe at a location distal from the location where the pressure change is caused, e.g. by means of a flow through element, such as a flow through valve, back flow preventing valve or such closing means. E.g. the pipe part between the injection point (e.g. flow through valve) and a (e.g. during flushing operated) closing means is flushed.

This pressure change for flushing is applied e.g. a short time (e.g. at the beginning and or end of flushing) or a long time (e.g. substantially the total time of flushing). The pressure is increased or decreased relative to the typical operating pressure of the sanitary system. Thus the pipe can be flushed with a flushing medium while within the pipe there is a higher or lower pressure compared to the environment.

The pressure change is preferably used for switching a closing means that is e.g. associated with a tap point. Thus care can be taken that the pipe or a part thereof, such as a tap point, obtains another action compared to typical operation. Thus, the pressure change can be applied as some kind of remote control. Electrical equipment is then superfluous.

A pressure change of short duration can e.g. be used for switching a closing means between two stable positions, e.g. open and closed. By maintaining the pressure change during the time of flushing, a valve with merely a single stable position can be kept in a second, unstable position, e.g. opened against a resilient or reset action.

The pressure change is preferably larger than a pressure fluctuation that happens during typical operation of the sanitary installation. Within houses and utility buildings the pressure of the to the public water delivery system connected sanitary installation is about 2.5 bar (atmospheric pressure head) during typical operating conditions and at street level. Pressure fluctuation for opening and closing of taps is typically no more than 5% of the typical operating pressure. The pressure change according to the invention is preferably at least 0.5 bar or 10% of the typical operating pressure. More preferably the pressure change is at least about 1 bar or 20%, more preferably at least about 1.5 bar or 30% of the typical operating pressure. To be able to flush effectively, the pressure in the pipe should be at least 1 bar (atmospheric pressure head).

Preferably a closing means is set such that it switches at about the pressure caused by the pressure change, or a pressure between the pressure caused by the pressure change and the typical operating pressure. E.g. taking a typical operating pressure of 2.5 bars as a starting point and a flushing pressure of 5 bars, the valve will switch at 4 bar.

Preferably one flushes with hot water, preferably higher than about 55 or 60 degrees Celsius to effectively kill bacteria. However, flushing with cold water, preferably below 25 degrees Celsius, can be a proper alternative. In stead of a chemical treatment, e.g. a chemical treatment can be carried out during flushing, e.g. with a solution of liquid and citric acid.

A generic elaboration of the invention, applied to a sanitary installation, is as follows: tap points (such as washstand, bath, sink taps and floater activated taps of toilet flushers), are provided with an own closing element designed such that during typical operation the tap point acts in the usual manner. This closing element is designed to switch, in reaction to sufficient change of the pipe/water pressure, between closing and flow through positions. To flush, the pipe pressure is changed, such that the closing elements switch into a different position. Thus the tap points will function differently from usual. E.g. it opens automatically or automatically connects two typically separated pipes (e.g. the hot and cold water pipe) in a single or both flow directions. Distal from the tap points the flushing medium is injected, e.g. in a pipe part common to all related tap points such as immediately downstream from the water meter. If flushing is completed, the closing elements are switched by the aid of a pressure change to recover the original situation.

The object is to treat an installation with a plurality of tap points integral, as much as possible automated/mechanised.

In the following, the invention is illustrated by way of non-limiting examples, referring to the enclosed drawing, schematically showing several pipe designs.

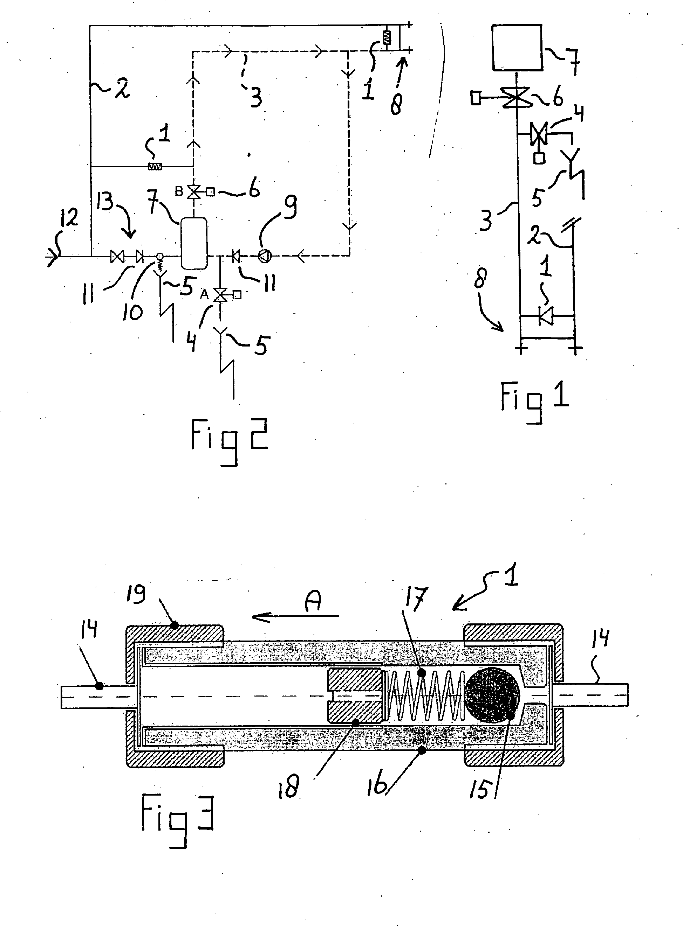

Fig. 1 is an example of flushing a hot water pipe with water from a cold water pipe. A flow through valve or check valve 1 is mounted between the cold water pipe 2 and hot water pipe 3 close to a mixer 8 functioning as a tap point. The flow through direction of the valve 1 (e.g. spring controlled check valve) is such that water can never flow from the hot to the cold water pipe. At equal water pressure in the pipes 2 and 3 (typical operating situation) valve 1 is closed. If the pressure in pipe 3 is sufficient lower than in pipe 2, valve 1 opens by overruling the bias and cold water can flow from pipe 2 into the hot water pipe.

Valve 1 has e.g. a biasing means such as a spring, with which the required pressure head between pipe 2 and 3 for opening/closing valve 1 can be adjusted.

To lower the pressure in pipe 3, upstream within pipe 3 a element, e.g. shutter 4, is mounted, yielding a pressure decrease within the pipe 3 downstream of it. Close to element 4, or integrated with it, there is a tap point 5 to drain the flushing water. Another element, e.g. shutter, 6 is provided to avoid water supply from the hot water supply 7 such as boiler or heater. The element 6 can be integrated with element 4, e.g. as three or multi way valve. Because of the pressure decrease by means of element 4, valve 1 automatically switches. The cold water flow from pipe 2 into pipe 3 and subsequently from tap point 5. The elements 4, 5 and/or 6 can be close to each other or spaced, e.g. element 6 can be upstream and element 4 downstream from valve 1. Valve 4 can be opened such that pipe 3 is flushed almost pressure less.

Based on the same principle, pipe 2 can be flushed with hot water from pipe 3. The flow through direction of valve 1 is then changed and the elements 4, 5 and 6 are associated with pipe 2.

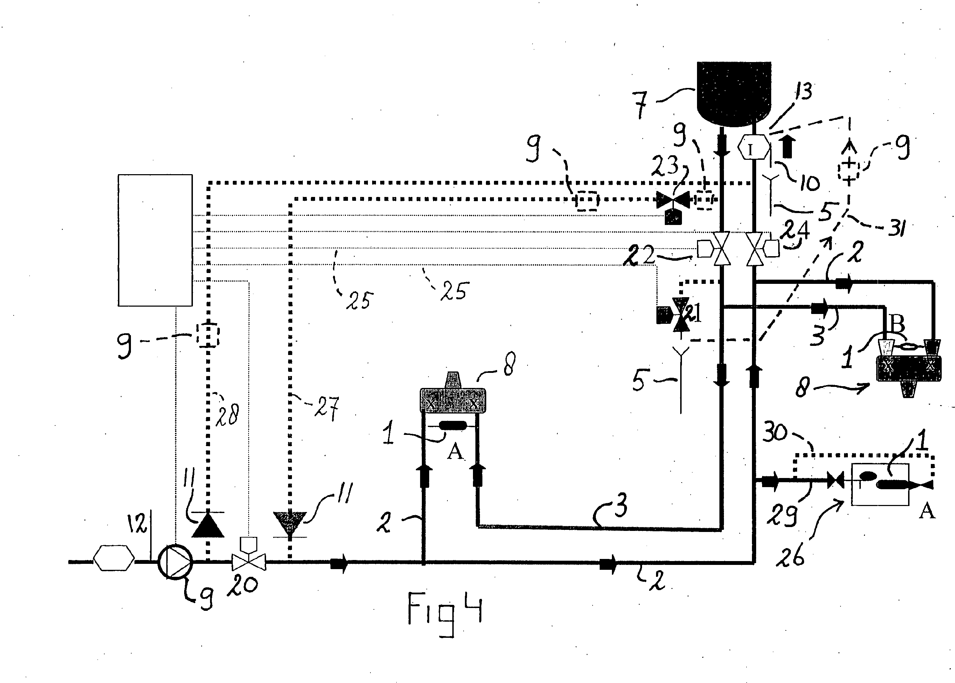

Fig. 2 and 3 show a system based on the principle of fig. 1 and an embodiment of the valve 1 in cross section.

Fig. 4-6 show a pipe design in three different operating modes, based on the principle of flushing with pressure increase. Illustrated are an electronic control unit to switch five valves and a pump (viz. thin lines), a sewer drain, a washstand mixer, boiler, batch mixer and toilet flusher. Interesting to mention is that at the two mixer taps, during flushing there is no water dispensing at these taps, which is safe with hot flushing. During flushing water is dispensed in the toiler flusher.

Based on this pipe design it is possible to make a design for flushing with cold water. Post rinsing is then superfluous. Also a variant is possible, wherein during flushing the water flows in closed circuit and is recirculated to the boiler. The invention is not limited to use of a single hot water supply for both typical use and flushing. Both functions can have a dedicated hot water supply. Fig. 4 will then be slightly different.

For each application of the invention it is preferred, that during flushing the pressure is substantially the same everywhere in the pipe that is flushed. This can e.g. be obtained by designing the switching valves that close to the tap points mutually connect the hot and cold water pipes during flushing, with a substantially smaller flow through area compared to the pipe, e.g. 10, 20 or 30% thereof, at the most.

While the drawing shows a single or some tap points, the invention is applicable to flushing a pipe to which many tap points are connected, in series and/or parallel.

In the embodiment of fig. 2, further illustrated are a circulation pump 9, a drain 10, check valves 11 and the connection 12 to the cold water system. Number 13 relates to the water inlet combination of the boiler 7. During typical operation, 6 is opened and 4 is closed. During flushing, 6 is closed and 4 opened, or both are closed. In both cases, 9 can operate. Arrows indicate the flow direction during flushing.

The valve 1 shown in fig. 3 comprises: connecting pipe 14; ball 15; body 16; biasing spring 17; screw 18 to adjust spring 17; nut 19. The flow through direction is indicated by arrow A.

During typical operation (fig. 4) of the system of fig. 4-6, pump 9 is de-activated, valves 20, 22 and 24 are opened, and valves 21 and 23 closed. If mixers 8 are opened and toilet reservoir 26 is flushed, the water flows according to the arrow direction through pipes 2, 3 and 29. No water flows through pipes 27, 28. Cold water is e.g. supplied at 2.5 bar at 12. Number 25 indicates control lines for switching components 9, 20, 21, 22, 23 and 24.

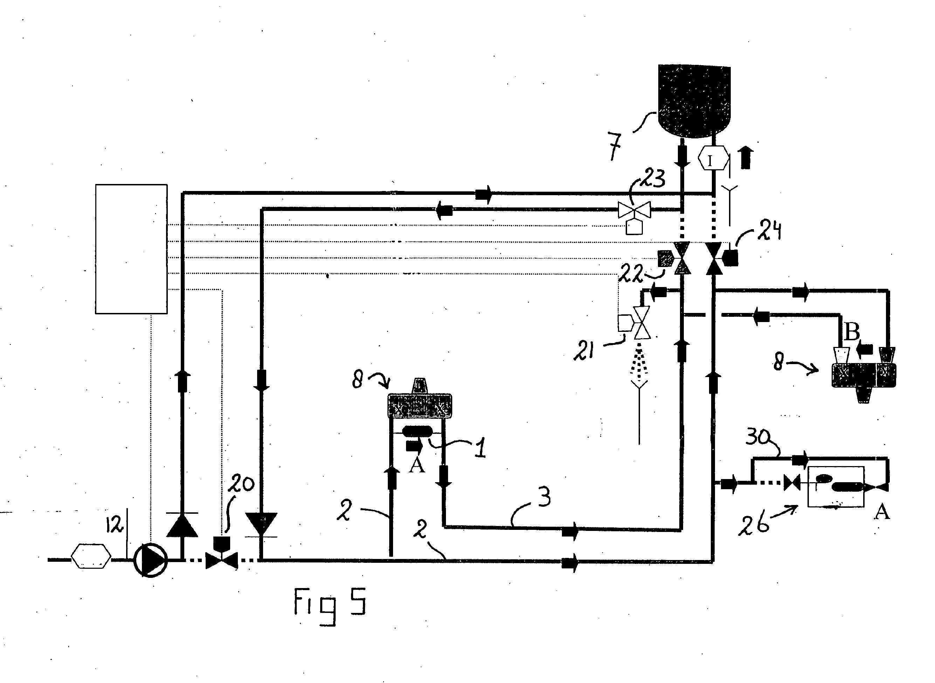

During flushing with hot water (fig. 5), pump 9 is activated, valves 20, 22 and 24 are closed, and valves 21 and 23 opened. Pump 9 increases the pressure to e.g. 5 bar. While mixers 8 and toiler reservoir 26 are closed, the water flows according to the direction of the arrow through the pipes 2, 3, 27, 28, 30 and through the valves 1 with which the hot and cold water pipe at the mixers 8 are short cut. Water is drained at valve 21 and into the toiler reservoir 26.

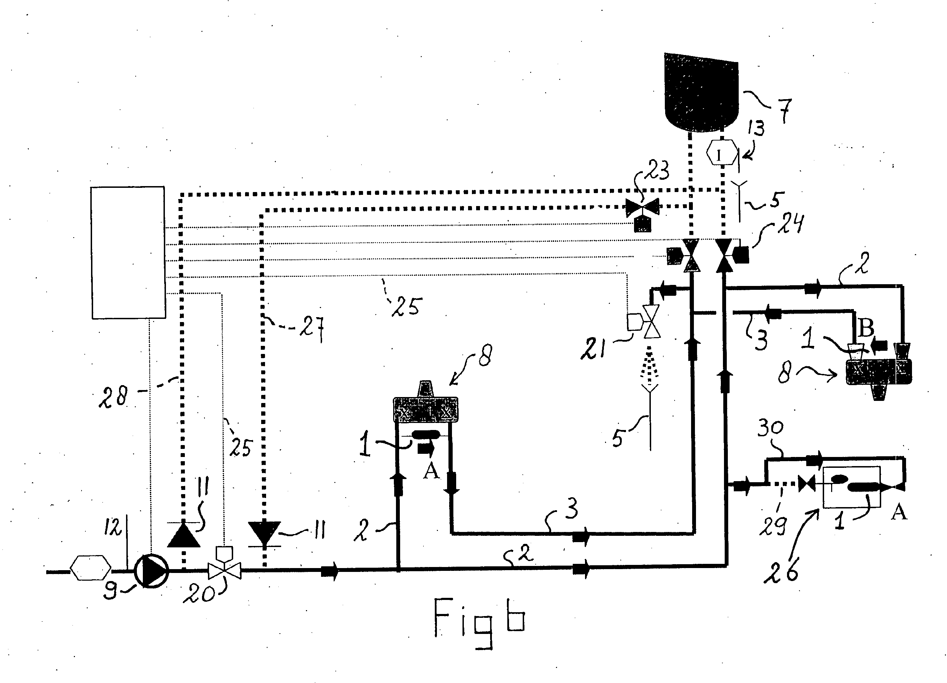

During post-rinsing with cold water (fig. 6), pump 9 supplies 5 bars, valves 20 and 21 are opened, and valves 23, 22 and 24 are closed. With closed mixers 8 and toiler reservoir 26, the water flows according to the arrows through the pipes 2, 3, 30 and through the valve 1 with which the hot and cold water pipe are short cut at the mixers 8. Water is drained at valve 21 and into the toilet reservoir 26. No water flows through the pipes 27, 28 and 29.

Alternatively, the pump 9 can be provided elsewhere, such as in pipe 27, 28 and/or 31, e.g. as shown in phantom in fig. 4. Location in pipe 27 and/or 31 is preferred.

During flushing with hot water (fig. 5) or post-rinsing with cold water (fig. 6), the water can recirculate. E.g. valve 21 is via pipe 31 (fig. 4) connected to the inlet combination 13 of boiler 7. Valve 21 can be provided as three way valve, for selectively closed, open to drain 5 or open to pipe 31.

The system of fig. 4-6 can also be operated as follows: During typical operation as fig. 4 shows. During flushing with hot water, as fig. 5 shows (components 8 and 26 closed), first with a lower pressure, compared to the pressure at 12 within pipe 3 while in pipe 2 a pressure as at 12. Pump 9 is preferably located within pipe 27 (upstream or downstream from valve 23) and/or pipe 31. Components 8 are then provided with a valve 1 that provided short cut between pipes 2, 3 by a pressure difference between said pipes. Pipe 30 contains the valve 1 of fig. 3 such that during this first phase no water flows through pipes 29 and 30. The water recirculates through pipe 31, possibly aided by pump 9 within pipe 31. After some time, e.g. when pipes 3 are sufficiently warmed, the water pressure in pipe 2 is increased with pump 9 (in e.g. pipe 27 and/or 31) compared to the pressure at 12. Valve 1 at component 26 then opens. Valves 1 at components 8 remain closed. The recirculation is maintained during this phase and hot water is drained via valve 1 of component 26. Preferably the pressure in pipe 3 remains lower than in pipe 2. Thereafter post-rinsing with cold water (fig. 6) with a pressure in pipe 2 equal 12, wherein valve 21 drains in 5, valve 1 of component 26 is closed and valves 1 of components 8 are opened, preferably with a pressure in pipe 3 lower than in 2. By selecting another type valve 1 for component 26, e.g. controlled by a short water pressure variation, hot water can flow through pipe 30 already during the first phase, such that the second phase is unnecessary.

Before the first phase a short draining of valve 21 into 5 can take place, with which the lowering of the pressure in pipe 3 is obtained.

The toilet reservoir 26 is an example of a water dispensing point with only cold water pipe, such that at said component no short cut can be made between cold and hot water pipe through valve 1. If valve 1 is conveniently selected, pipes 29 and 30 can be integrated. Pipe 29 is floater controlled known as such. If component 26 is a cold water tap, such as outdoor tap, pipes 29 and 30 are integrated and opened valve 1 will let hot water flow from the tap. To avoid this, component 26 can be connected to a return pipe, connected to pipe 3, wherein opened valve 1 short cuts the return pipe with pipe 29 or 30.

The procedures disclosed on the basis of fig. 4-6 can also be applied to differently designed systems. E.g. a system is feasible wherein components 26 are not treated according to the invention.

## Claims

1. Flushing method in a water pipe system, comprising pipes for hot (3) and cold (2, 29) water, connected to tap points (8, 26) to dispense hot and/or cold water, and provided with means for flushing a pipe (2, 3, 29) with a treatment medium, wherein said means are designed to make said flushing possible by applying a pressure change in the water pipe system or part of it, preferably of at least 0.5 bar.

2. Method according to claim 1, with the system comprising a means (1) reacting to the pressure change, e.g. a valve, designed to short circuit the pipes (2, 3) or draining through a tap point (26), and/or a decreased, e.g. to 10%, flow trough area which is activated during flushing.

3. Method according to claim 1 or 2, wherein the system comprises a means (4, 9), e.g. valve, drain or pump, to provide a pressure increase or decrease.

4. Method according to any of claims 1-3, wherein the means (4, 9) is located upstream or downstream at a distance of the means (1), and/or injection location of treatment medium.

5. Method according to any of claims 1-4, the system comprising a cold water supply (12) and a hot water supply (7), valves (4, 6, 20, 21, 22, 23, 24), pipes (27, 28, 29, 30), connected to switch between a typical operating position wherein the pipes (27, 28, 29) are out of use and a flushing position wherein fluid flow through the pipes (28, 29, 30) and pipes (2, 3).

6. Method according to any of claims 1-5, and the system is designed to recirculate fluid during flushing, e.g. by means of a pipe (31) and/or shutter (21), preferably through the hot water supply (7).

7. Method according to any of claims 1-6, and the system is designed to flush and post-rinse, wherein possibly flushing 5 is carried out first at the one pressure level and then another, different, preferably higher, pressure level, e.g. to activate additional tap points (26), e.g. for draining.

8. Method according to any of claims 1-7, and the system is designed to flush and/or post-rinse at a pressure within the pipe (3) different from the pressure within pipe (2), preferably a pressure within the pipe (3) lower than pipe (2), e.g. by draining.

9. Method according to any of claims 1-8, and the system designed to drain at the beginning of flushing, preferably from pipe (3), e.g. by a pressure decrease, to subsequently recirculate, to subsequently change the pressure, preferably increase it, to activate additional tap points (26), e.g. by draining.

10. Method according to any of claims 1-9, and the system designed such that in the flush and/or post-rinse mode, the flow direction through the pipe (2) and/or (3) is opposite.
